# EUROPÄISCHE PATENTSCHRIFT

(11) **EP 3 908 648 B1**
(45) Veröffentlichungstag und Bekanntmachung des Hinweises auf die Patenterteilung: **28.01.2026**
(21) Anmeldenummer: 19832932.8
(22) Anmeldetag: 19.12.2019
(51) Int. Cl.: C12M 1/00, C12M 1/12

(54) **BIOREAKTOR MIT FILTERTASCHE UND VERFAHREN ZU DEREN HERSTELLUNG**
BIOREACTOR COMPRISING A FILTER POCKET, AND METHOD FOR PRODUCING SAME
BIORÉACTEUR DOTÉ D'UNE POCHE DE FILTRE ET PROCÉDÉ POUR LEUR PRÉPARATION

(30) Priorität: 09.01.2019 DE 102019100434
(43) Veröffentlichungstag der Anmeldung: 17.11.2021
(73) Patentinhaber: Sartorius Stedim Biotech GmbH, 37079 Göttingen (DE)
(72) Erfinder: BLUMA, Arne, 37079 Goettingen (DE); FAULSTICH, Franziska, 37079 Goettingen (DE); TUNZINI, Fabian, 8317 Tagelswangen (CH); DELESSERT, Rachel, 8317 Tagelswangen (CH); TUOR, Darius, 8317 Tagelswangen (CH)
(74) Vertreter: Novagraaf International SA
(86) Internationale Anmeldenummer: PCT/EP2019/086465
(87) Internationale Veröffentlichungsnummer: WO 2020/144049

(56) Entgegenhaltungen:
- EP-A2- 1 132 460
- EP-B1- 2 268 788
- US-A1- 2014 011 270
- US-A1- 2014 287 512
- US-A1- 2018 346 864

## Beschreibung

Die Erfindung betrifft einen Bioreaktor zur Kultivierung von Mikroorganismen und Zellen tierischer oder pflanzlicher Herkunft. Die Erfindung betrifft ferner ein Verfahren zur Herstellung einer Filtertasche eines solchen Bioreaktors.

Aus EP 2 268 788 B1 ist ein als flacher Einweg-Beutel ausgebildeter Bioreaktor bekannt, bei dem wenigstens eine Filtertasche an der flüssigkeitsbenetzten Innenfläche einer Wand des Bioreaktors fixiert ist. Die Filtertasche ist durch Schweißen der Ränder eines hydrophilen Filtermediums auf die Innenwand des Bioreaktors gebildet, sodass die durchgehende Schweißnaht einen Sammel- und/oder Verteilerraum zwischen dem Filtermedium und der Innenwand des Bioreaktors begrenzt. Der Sammel-/Verteilerraum ist mit mindestens einem Anschluss für ein Zu- und/oder Abführen von Medien kommunizierend verbunden. Im Sammel-/Verteilerraum kann ein Abstandshalter in Form eines Stützgewebes vorgesehen sein, der das Anhaften des Filtermediums an der Bioreaktorwand und damit das Kollabieren des Sammel-/Verteilerraums verhindert. Ein solcher Bioreaktor ist zur Perfusion geeignet, insbesondere als Einweg-Bioreaktor zur Verwendung auf einer Kippvorrichtung ("rocking motion bag" oder "rocker bag") und ist im Handel erhältlich in der Größe von 2 bis 50 Litern (Totalvolumen, entspricht bis zu 25 Litern Arbeitsvolumen). Für größere Volumina ergibt sich aufgrund der steigenden mechanischen Belastung, die unter anderem auf die Filtertasche wirkt, die Notwendigkeit, einen Bioreaktor bereitzustellen, der für größere Volumina geeignet ist.

Die US 2018/0346864 A1 zeigt einen Beutelbioreaktor zur Kultivierung von Zellen mit einem Filter im Reaktorinneren und einer mit Befestigungsmitteln an der Innenwand des Beutels angebrachten Filterhaltevorrichtung. An der Filterhaltevorrichtung sind Abstandsmittel in Form von Rippen vorgesehen, um den Filter in einem Abstand von der Innenwand des Bioreaktors zu halten. Die Filterhaltevorrichtung umfasst einen Anschluss, der um eine Öffnung in der Filterhaltevorrichtung herum angeordnet ist. Die Öffnung stellt eine Fluidverbindung mit den Zwischenräumen zwischen den Rippen her und ist so angeordnet, dass sie mit einer Schlauchleitung zur Entnahme von Filtrat verbunden werden kann.

In der US 2014/0287512 A1 ist ein Beutelbioreaktor zur Perfusionskultivierung von Zellen mit einer Kammer im Inneren des Beutels gezeigt. Die Kammer ist durch ein Filtermaterial begrenzt und so an der Innenseite einer Wand des Beutels befestigt, dass es sich beabstandet zur Wand befindet. Eine Strömungsverbindung der Kammer mit einem Anschluss in einer Beutelwand außerhalb der Kammer kann durch einen Schlauch im Inneren des Beutels gebildet sein. Es kann aber auch ein Anschluss in der Beutelwand an einer Stelle vorgesehen sein, wo die Beutelwand als Außenwand der Kammer dient.

Aufgabe der Erfindung ist es, einen zur Perfusion geeigneten Bioreaktor auch für große Volumina bereitzustellen, insbesondere in der Größenordnung von 100 oder 200 Litern (Totalvolumen) oder auch darüber.

Gelöst wird diese Aufgabe durch einen Bioreaktor mit den Merkmalen des Anspruchs 1 sowie durch ein Verfahren mit den Merkmalen des Anspruchs 10. Vorteilhafte und zweckmäßige Ausgestaltungen des erfindungsgemäßen Bioreaktors und des erfindungsgemäßen Verfahrens sind in den zugehörigen Unteransprüchen angegeben.

Der erfindungsgemäße Bioreaktor zur Kultivierung von Mikroorganismen und Zellen tierischer oder pflanzlicher Herkunft, vorzugsweise ein Einweg-Bioreaktor, umfasst eine auf der Innenfläche einer flexiblen Wand des Bioreaktors angeordnete Filtertasche, die außenseitig durch die Wand des Bioreaktors und innenseitig durch eine Filtertaschenwand begrenzt ist. Die innenseitige Filtertaschenwand ist zumindest teilweise durch ein Filtermedium gebildet. In der Filtertasche ist zwischen der Wand des Bioreaktors und dem Filtermedium ein Abstandshalter angeordnet. Gemäß der Erfindung weist der Abstandshalter wenigstens eine Durchgangsöffnung auf. Ein Verbindungsabschnitt des Filtermediums ragt durch die Durchgangsöffnung hindurch und ist direkt mit der Wand des Bioreaktors verbunden.

Die Erfindung basiert auf mehreren Erkenntnissen. Zunächst hat sich bei Versuchen gezeigt, dass bei Bioreaktoren mit einem Totalvolumen von 100 Litern oder mehr eine Randschweißung des Filtermediums nicht mehr ausreichend ist, da sich der Rand des Filtermediums von der Bioreaktorwand lösen kann. In einem solchen Fall wäre die Funktion der Filtertasche nicht mehr gewährleistet, da auch unfiltriertes Medium aus dem Bioreaktor in die Filtertasche gelangen könnte. Der Grund hierfür ist die große Flüssigkeitsmenge und deren Gewicht im Bioreaktor, d. h es wirken schon alleine aufgrund des hydrostatischen Drucks entsprechend große Kräfte auf die Filtertasche. Im Falle der Verwendung des Bioreaktors auf einer Kippvorrichtung erhöht sich die Belastung der Filtertasche zusätzlich aufgrund des schwappenden Mediums und der dadurch bedingten Kraftstöße und Reibungskräfte infolge der Relativbewegungen.

Außerdem wurde erkannt, dass sich die bevorzugten Materialien des Filtermediums, des Abstandshalters und der Bioreaktorwand nicht ohne Weiteres miteinander verbinden lassen. Das Herstellen eines Verbunds aus Filtermedium, Abstandshalter und Bioreaktorwand durch Schweißen ist schon aufgrund der deutlich unterschiedlichen Schmelzpunkte der bevorzugten Materialien problematisch, sofern ein Verschweißen dieser Materialien überhaupt möglich ist. Ähnliches gilt auch für ein Verkleben der Komponenten oder andere Verbindungsarten.

Die Erfindung überwindet diese Probleme, indem nur das Filtermedium mit der Wand des Bioreaktors verbunden wird. Zusätzlich zu einer umlaufenden, fluiddichten Randverbindung des Filtermediums mit der Bioreaktorwand, wie sie aus dem Stand der Technik bekannt ist, sieht die Erfindung zur weiteren Stabilisierung der Filtertasche zusätzlich mindestens eine punktuelle Verbindung zwischen dem Filtermedium und der Bioreaktorwand im mittleren Bereich der Filtertasche (also nicht in deren Randbereich) vor, ohne dass der dazwischen angeordnete Abstandshalter an dieser mindestens einen Verbindung partizipiert, d. h. es ist keine unmittelbare Verbindung zwischen dem Abstandshalter und dem Filtermedium bzw. zwischen dem Abstandshalter und der Bioreaktorwand vorgesehen. Dies wird erfindungsgemäß dadurch erreicht, dass der Abstandshalter wenigstens eine Durchgangsöffnung aufweist und ein Verbindungsabschnitt des Filtermediums, der durch die Durchgangsöffnung hindurchragt, direkt mit der Wand des Bioreaktors verbunden ist. Auf diese Weise ist es möglich, den Abstandshalter bei der Schaffung weiterer Befestigungsstellen für die Filtertasche außen vor zu lassen, d. h. nur die Materialien des Filtermediums und der Bioreaktorwand gehen eine Verbindung ein.

Ein besonders vorteilhafter Nebeneffekt der erfindungsgemäßen zusätzlichen Befestigung der Filtertasche ist die fixe Positionierung des Abstandshalters in der Filtertasche durch die Verbindungsabschnitte des Filtermediums, die durch die Durchgangsöffnungen des Abstandshalters hindurchragen. Diese fixe Positionierung kommt ohne Schweißen, Kleben oder eine andere Fügetechnik dank der erfindungsgemäßen Gestaltung automatisch zustande. Dies ist insbesondere vorteilhaft, da sich der Abstandshalter während der Benutzung nicht mechanisch verformt, aufrollt, auffaltet o.ä.

Gemäß der bevorzugten Ausführungsform der Erfindung sind mehrere Durchgangsöffnungen vorgesehen, die vorzugsweise regelmäßig über den Abstandshalter verteilt angeordnet sind. Dementsprechend ragt durch jede Durchgangsöffnung im Abstandshalter ein Verbindungsabschnitt des Filtermediums und ist direkt mit der Wand des Bioreaktors verbunden. Durch eine geschickte Wahl von Anzahl, Anordnung, Größe und Form der punktartigen Verbindungen lässt sich ein optimales Verhältnis zwischen guter Stabilität der Filtertasche, möglichst geringer Beeinträchtigung der Verteilung des gefilterten Mediums in der Filtertasche und eine größtmögliche durch Strömung frei nutzbare Membranfläche, bei der die geschweißte Fläche im Vergleich zur Gesamtmembranfläche so gering wie möglich ist, erreichen.

In diesem Zusammenhang ist eine Ausführungsform vorteilhaft, bei der die Filtertasche frei von vollständig oder teilweise abgetrennten Kammern ist. Das bedeutet, dass die zusätzlich an der Bioreaktorwand fixierten Verbindungsabschnitte des Filtermediums hinsichtlich Anzahl, Anordnung, Größe und Form so gewählt sind, dass sich in der Filtertasche keine Toträume oder dergleichen bilden. Insbesondere ist es nicht vorteilhaft, durch die Anordnung der punktförmigen Verbindungsabschnitte ganz oder nahezu geschlossene Verbindungslinien zu bilden, die zu vollständig oder nahezu vollständig geschlossenen Kammern innerhalb der Filtertasche führen würden.

Die Erfindung eignet sich im Hinblick auf Materialien, die für bestimmte Perfusionsprozesse bevorzugt sind, insbesondere für einen Bioreaktor, dessen Wand unter anderem aus Polyethylen gebildet ist, in Kombination mit einem Filtermedium in Form einer Mikrofiltrationsmembran, die zumindest teilweise vorzugsweise aus aliphatischen Polyamiden, Polysulfonen und Polyethersulfone, Polyester, Polyvinylidenhalogeniden, Acrylpolymeren, Acryl-Copolymeren und Zelluloseestern, besonders bevorzugt aus Polyethersulfon gebildet ist. Die Mikrofiltrationsmembran ist auf wenigstens einer ihrer beiden Seiten mit einem porösen Flächengebilde verbunden, insbesondere einem Polypropylen/Polyethylen-Polyethylenterephthalat-Polypropylen/Polyethylen Laminat. Diese Materialien lassen sich gut miteinander verbinden, insbesondere durch Schweißen. Der nicht an der Verbindung beteiligte Abstandshalter besteht vorzugsweise aus Polyethylenterephthalat, das sich nicht oder nur unzureichend mit den zuvor genannten Materialien verbinden lässt. Prinzipiell sind weitere Abstandshalter aus mechanisch und chemisch inerten Materialien möglich, deren Quellungs- und Schrumpfungsgrad so gering ist, dass er die Funktion der Membran nicht negativ beeinflusst.

Um eine einfaches Zuführen und/oder Abführen von Medium aus der Filtertasche zu ermöglichen, ist in das Filtermedium vorzugsweise ein Anschlussstück eingesetzt, an das eine Schlauchleitung anschließbar ist, die aus dem Bioreaktor herausführt.

Die Vorteile der Erfindung kommen am besten zum Tragen bei Bioreaktoren mit einem maximalen totalen Volumen von wenigstens 50 Litern, vorzugsweise 100 Litern, 200 Litern und bis zu 1.000 Litern, insbesondere bei Einweg-Bioreaktoren dieser Größe, die zur Verwendung auf einer Kippvorrichtung vorgesehen sind.

Die Erfindung schafft auch ein Verfahren zur Herstellung einer Filtertasche in einem Bioreaktor zur Kultivierung von Mikroorganismen und Zellen tierischer oder pflanzlicher Herkunft mit folgenden Schritten: Bereitstellen einer flexiblen Wand für die Ausbildung eines Bioreaktors, eines Abstandshalters und einer Filtertaschenwand, die zumindest teilweise durch ein Filtermedium gebildet ist, wobei die Filtertaschenwand eine größere Flächenausdehnung als der Abstandshalter hat; Bilden wenigstens einer Durchgangsöffnung im Abstandshalter, die den Abstandshalter vollständig durchdringt; Platzieren des Abstandshalters auf der Innenfläche der unteren Wand des Bioreaktors; Platzieren der Filtertaschenwand auf dem Abstandshalter; umlaufendes Fixieren des über den Abstandshalter überstehenden Rands der Filtertasche an der Wand des Bioreaktors; Durchführen eines Verbindungsabschnitts des Filtermediums durch die Durchgangsöffnung; und Fixieren des Verbindungsabschnitts des Filtermediums direkt an der Wand des Bioreaktors.

Das erfindungsgemäße Herstellungsverfahren macht sich die Erkenntnis zunutze, dass der Abstandshalter problemlos "gelocht" werden kann, und dass später durch die so entstandenen Durchgangsöffnungen hindurch das Filtermedium direkt mit der Bioreaktorwand verbunden werden kann (vorzugsweise im selben Schritt), ohne dass der Abstandshalter selbst eine direkte Verbindung mit dem Filtermedium oder der Bioreaktorwand eingehen muss. Dabei sollte jeweils der Querschnitt des erzeugten Lochs im Abstandshalter etwas größer sein als der spätere Verbindungsabschnitt, d. h. als der Punkt- bzw. Flächenbereich der direkten Verbindung zwischen Filtermedium und Bioreaktorwand. Dadurch ist sichergestellt, dass beim Verbinden des Filtermediums mit der Bioreaktorwand, insbesondere durch Schweißen, nicht versehentlich das Material des Abstandshalters mitverschweißt wird. Die Lochgröße gewährleistet damit die Festigkeit der Verbindungspunkte bzw. - bereiche, und in der Anwendung können keine Löcher entstehen.

Nach der erfindungsgemäßen Herstellung der Filtertasche wird der Bioreaktor fertiggestellt. Dabei ist es grundsätzlich unerheblich, ob der Bioreaktor im Wesentlichen alleine aus der flexiblen Wand mit der Filtertasche gebildet wird, z. B. durch Falten der Wand und Verbinden der aufeinanderliegenden freien Randabschnitte, oder ob eine oder mehrere weitere Wände zu einem Bioreaktor miteinander verbunden werden.

Das Fixieren des wenigstens einen Verbindungsabschnitts des Filtermediums an der Wand des Bioreaktors erfolgt am besten durch Punktschweißen. Geeignete Anlagen sind auf dem Markt verfügbar, und die Verbindungstechnik als solche ist erprobt und etabliert.

Alternativ kann das Fixieren des wenigstens einen Verbindungsabschnitts des Filtermediums an der Wand des Bioreaktors auch durch Kleben erfolgen.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 einen Abstandshalter für eine Filtertasche in einem erfindungsgemäßen Bioreaktor nach einer ersten Ausführungsform;
- Figur 2 ein Filtermedium zur Ausbildung einer Filtertasche in einem erfindungsgemäßen Bioreaktor nach der ersten Ausführungsform;
- Figur 3 eine fertige Filtertasche in einem erfindungsgemäßen Bioreaktor nach der ersten Ausführungsform;
- Figur 4 einen Abstandshalter für eine Filtertasche in einem erfindungsgemäßen Bioreaktor nach einer zweiten Ausführungsform;
- Figur 5 ein Filtermedium zur Ausbildung einer Filtertasche in einem erfindungsgemäßen Bioreaktor nach der zweiten Ausführungsform; und
- Figur 6 eine fertige Filtertasche in einem erfindungsgemäßen Bioreaktor nach der zweiten Ausführungsform.

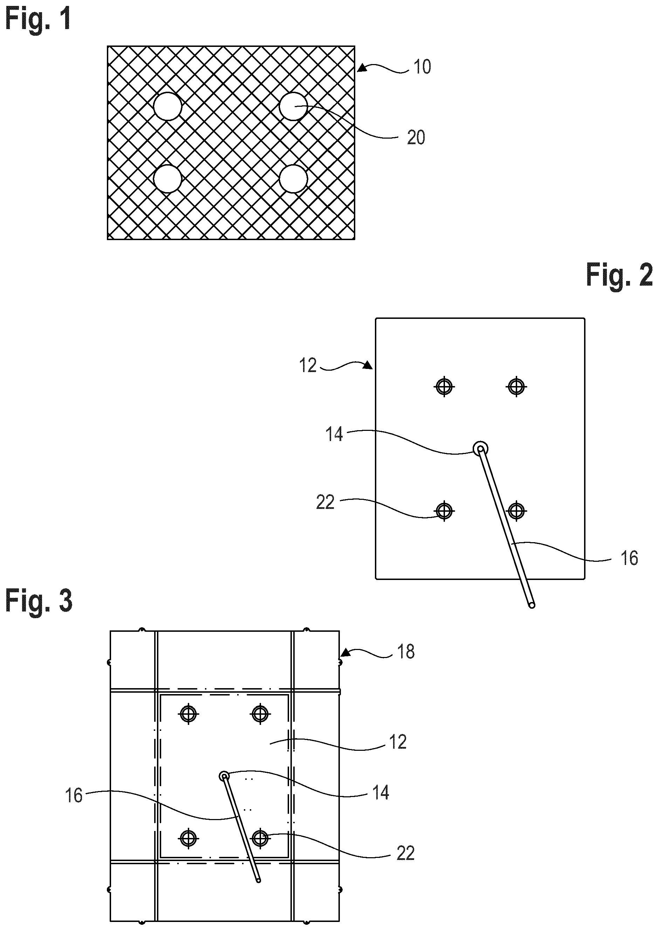

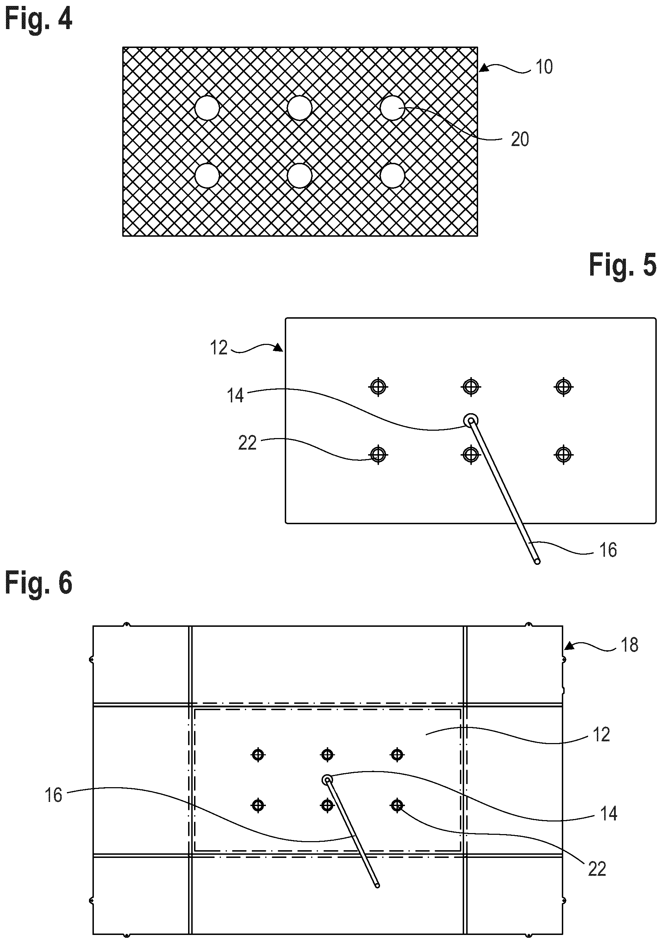

Die Figuren zeigen beispielhaft, wie in einem großen Bioreaktor eine Filtertasche gebildet werden kann. Konkret beziehen sich die Figuren 1 bis 3 auf eine erste Ausführungsform eines Bioreaktors mit einem Totalvolumen von 100 Litern (empfohlenes Arbeitsvolumen: 50 Liter), die Figuren 4 bis 6 auf eine zweite Ausführungsform eines Bioreaktors mit einem Totalvolumen von 200 Litern (empfohlenes Arbeitsvolumen: 100 Liter). Die nachfolgende Beschreibung gilt grundsätzlich für beide Ausführungsformen. Die Unterschiede zwischen den beiden Ausführungsformen werden am Ende separat erläutert.

In den Figuren 1 und 4 ist jeweils ein Zuschnitt eines grobmaschigen technischen Textils gezeigt, das in der fertigen Filtertasche als Abstandshalter 10 dient. Das Textil ist vorzugsweise aus PET-Fasern (Polyethylenterephthalat) hergestellt, kann aber auch auf andere Weise und/oder aus einem anderen flexiblen, bruch- und knickfesten Material gebildet sein. Wichtig ist, dass der Abstandshalter 10 in der Filtertasche vom filtrierten (zellfreien) Medium durchströmt werden kann - also insbesondere nicht massiv ist -, aber dennoch stabil genug ist, um ein Kollabieren des in der Filtertasche definierten Raums zu verhindern. Außerdem muss gewährleistet sein, dass aus dem Abstandshalter 10 bestimmte Bereiche herausgetrennt werden können. Auf diese Eigenschaft des Abstandshalters 10 wird später noch genauer eingegangen.

Die Figuren 2 und 5 zeigen jeweils ein Filtermedium 12, mit dem die Innenwand der Filtertasche im Bioreaktor gebildet wird. Das Filtermedium 12 ist für Zellen undurchlässig, für sonstige Inhaltsstoffe der Zellsuspension jedoch gut durchlässig. Hierfür geeignet sind Mikrofiltrationsmembranen (nachfolgend nur noch als Membranen bezeichnet) mit einer effektiven Porengröße von maximal 10 µm, z. B. eine bei Benetzung nicht quellende, hydrophile Polyethersulfonmembran (PESU) mit einer Porengröße von 1,2 µm. Die Poren sind in jedem Fall kleiner als die Maschen des Abstandshalters 10.

Eine oder beide Seiten der Membran können mit einem porösen Flächengebilde adhäsiv verbunden sein, z. B. einem stabilen Kernmantelvlies mit einem PP/PE-PET-PP/PE Schichtaufbau (Polypropylen/Polyethylen-Polyethylenterephthalat-Polypropylen/Polyethylen Laminat).

In das Filtermedium 12 ist ein Anschlussstück 14 eingearbeitet, beispielsweise eine Schlaucholive, welches das Filtermedium 12 vollständig durchdringt. Wenigstens auf einer Seite des Filtermediums 12 ist eine Schlauchleitung 16 an das Anschlussstück 14 anschließbar.

Nachfolgend wird beispielhaft die Herstellung einer Filtertasche mithilfe des als Filtertaschenwand dienenden Filtermediums 12 und des Abstandshalters 10 im Inneren eines flexiblen Einweg-Bioreaktors beschrieben, dessen Wand 18 vorzugsweise aus PE (Polyethylen) gebildet ist.

Der Abstandshalter 10 wird an vorgegebenen Stellen "gelocht", d. h. es werden an diesen Stellen Durchgangsöffnungen 20 gebildet, die den Abstandshalter 10 vollständig durchdringen. Die Durchgangsöffnungen 20 können auf verschiedene Weise, z. B. durch Stanzen, und in grundsätzlich beliebiger Form, bevorzugt jedoch mit kreisrundem Umfang, hergestellt werden. In den Figuren 1 und 4 ist der Abstandshalter 10 bereits mit vier bzw. sechs Durchgangsöffnungen 20 versehen.

Vor der Fertigstellung des Bioreaktors werden auf die Innenseite der Bioreaktorwand 18, die bei Gebrauch des Bioreaktors dem Kulturmedium zugewandt ist, an der Stelle, an der die Filtertasche gebildet werden soll, zunächst der Abstandshalter 10 und darüber das größere, den Abstandshalter 10 an allen Seiten überragende Filtermedium 12 gelegt.

Anschließend wird der Rand der Filtertaschenwand, die beim hier beschriebenen Ausführungsbeispiel alleine aus dem Filtermedium 12 gebildet ist, vollständig umlaufend mit der Bioreaktorwand 18 fluiddicht verbunden, insbesondere durch Schweißen. Unter einer solchen fluiddichten Verbindung ist auch ein indirektes Anbringen der Filtertaschenwand bzw. des Filtermediums 12 an der Bioreaktorwand 18 mit dazwischen angeordnetem Material zu verstehen. Eine indirekte fluiddichte Verbindung kann gegebenenfalls vorteilhaft sein, wenn sich dadurch eine verbesserte Haftung und/oder Dichtigkeit im Vergleich zu einer direkten Verbindung erreichen lässt. Die dadurch gebildete, noch nicht fertige Filtertasche ist somit außenseitig durch die Bioreaktorwand 18 und innenseitig (zumindest teilweise) durch das Filtermedium 12 begrenzt. Der Abstandshalter 10 ist an diesem Verbindungsschritt nicht beteiligt, außer dass er durch die Fixierung des Rands des Filtermediums 12 an der Bioreaktorwand 18 in der dadurch gebildeten Filtertasche "gefangen" ist.

Danach werden in einem weiteren Verbindungsschritt zusätzliche punktartige Verbindungen zwischen dem Filtermedium 12 und der Bioreaktorwand 18 hergestellt. Hierzu werden entsprechende Verbindungsabschnitte 22 des Filtermediums 12 durch die Durchgangsöffnungen 20 des Abstandshalters 10 hindurch gedrückt und direkt an der Bioreaktorwand 18 fixiert. Dies ist z. B. durch Verkleben oder lokales Erhitzen und Verschmelzen möglich, insbesondere mit einer Punktschweißanlage. Auch an diesem Verbindungsschritt ist der Abstandshalter 10 nicht unmittelbar beteiligt, d. h. der Abstandshalter 10 selbst geht weder eine Verbindung mit dem Filtermedium 12 noch mit der Bioreaktorwand 18 ein. Die Beweglichkeit des Abstandshalters 10 in der Filtertasche ist durch die Punktverbindungen jedoch stark eingeschränkt oder ganz unterbunden, was vorteilhaft und deshalb erwünscht ist.

Bei einem verhältnismäßig dicken Abstandshalter 10 und/oder einem verhältnismäßig unflexiblen Filtermedium 12 können auf der der Bioreaktorwand 18 zugeordneten Seite des Filtermediums 12 vorstehende Nippel vorgesehen sein, deren Anordnung auf die Anordnung der Durchgangsöffnungen 20 im Abstandshalter 10 abgestimmt sind. Das Filtermedium 12 wird dann vor dem Fixieren so auf den Abstandshalter 10 gelegt, dass sich die Nippel in die Durchgangsöffnungen 20 hinein bzw. durch diese hindurch erstrecken, um das Fixieren des Filtermediums 12 an diesen Stellen zu erleichtern. Derartige Nippel können zusätzlich zu den Nippeln des Filtermediums oder anstelle dieser Nippel auch auf der Bioreaktorwand 18 vorgesehen sein.

Die genaue Form der Punktverbindungen ist nicht von besonderer Bedeutung. Beispielsweise können mit einem erhitzten Zylinderrohr ringförmige Befestigungsstellen gebildet werden.

Von Bedeutung ist hingegen, dass die Punktverbindungen nicht im Randbereich, sondern in einem mittleren Bereich des Filtermediums 12 angeordnet sind. Dadurch ist nicht nur gewährleistet, dass die Verbindung zwischen dem Filtermedium 12 und der Bioreaktorwand 18 erheblich verstärkt ist, sondern auch, dass der Abstandshalter 10 in seiner Lage fixiert ist, ohne dass er an der Verbindung zwischen dem Filtermedium 12 und der Bioreaktorwand 18 direkt beteiligt ist. Die einzelnen Punktverbindungen sorgen zudem dafür, dass in der Filtertasche keine Kammern oder sonstige Toträume abgetrennt bzw. abgegrenzt sind. Das Medium kann vielmehr jeden Bereich der Filtertasche erreichen.

Wie in den Figuren 3 und 6 gezeigt, ist nach Beendigung der beiden oben beschriebenen Verbindungsschritte, die prinzipiell auch in umgekehrter Reihenfolge durchgeführt werden können, im Bioreaktor eine perfusionstaugliche Filtertasche gebildet, die extrem robust ist. Anschließend wird der Bioreaktor in an sich bekannter Weise fertiggestellt.

Die innenseitige Filtertaschenwand kann auch nur teilweise aus dem Filtermedium 12 gebildet sein, d. h. sie muss nicht zwingend vollständig aus dem Filtermedium 12 bestehen.

Die unterschiedlichen Materialien des Filtermediums 12, des Abstandshalters 10 und der Bioreaktorwand 18 sind so gewählt, dass der Abstandshalter 10 - insbesondere während des Gebrauchs des Bioreaktors - weder am Filtermedium 12 noch an der Bioreaktorwand 18 klebt.

An das von der Filtertasche abgewandte Anschlussstück 14 im Filtermedium 12 lässt sich eine Schlauchleitung 16 anschließen, die aus dem Bioreaktor herausgeführt werden kann und insbesondere zur Abfuhr von Medium aus der Filtertasche vorgesehen ist. Grundsätzlich sind auch andere Ausgestaltungen möglich. Wesentlich ist, dass die Filtertasche über wenigstens einen Fluidanschluss verfügt, über den eine separate Strömungsverbindung realisierbar ist, die vom Medium im Bioreaktor außerhalb der Filtertasche isoliert ist, damit sich das aus der Filtertasche abzuführende Medium nicht mit dem restlichen Medium im Bioreaktor vermischt.

Wie bereits erwähnt beziehen sich die Figuren 1 bis 3 auf eine erste Ausführungsform eines Bioreaktors mit einem Totalvolumen von 100 Litern und die Figuren 4 bis 6 auf eine zweite Ausführungsform eines Bioreaktors mit einem Totalvolumen von 200 Litern. Im Unterschied zur ersten Ausführungsform sind bei der zweiten Ausführungsform nicht vier, sondern sechs regelmäßig angeordnete Punktverbindungen vorgesehen. Die konkrete Anzahl, Anordnung, Form und Größe der Punktverbindungen sind aber weder für die oben beschriebenen noch für andere Ausführungsformen einschränkend zu verstehen, sondern sind grundsätzlich variabel.

Größe, Form und Lage der Filtertasche im Bioreaktor können je nach Anwendung weitgehend frei gewählt werden. Beispielsweise sind eine sehr langgestreckte Filtertasche oder sogar eine vollständig über den Innenumfang des Bioreaktors umlaufende Filtertasche denkbar. In letzterem Fall wäre unter einer "umlaufenden" Randverbindung der Filtertaschenwand eine Verbindung der beiden Längsränder der ringförmigen Filtertaschenwand mit der Bioreaktorwand 18 zu verstehen.

Allgemein sollte die konkrete Ausgestaltung der Filtertasche folgende Anforderungen und Kriterien erfüllen:
Einerseits muss die Stabilität der Filtertasche für den jeweils vorgesehenen Verwendungszweck jederzeit sichergestellt sein. Andererseits sollte aber die durch Strömung nutzbare Membranfläche des Filtermediums 12 möglichst wenig eingeschränkt sein, d. h. die aufgrund der Punktverbindungen nicht nutzbare Filterfläche sollte so klein wie möglich sein. Außerdem sollen die Punktverbindungen die Verteilung des Mediums in der Filtertasche möglichst wenig beeinträchtigen. Auch die Funktion des Anschlussstücks 14 muss gewährleistet sein.

Das beschriebene Konzept der Herstellung robuster Filtertaschen ist bei Bioreaktoren der Größen 100 Liter und 200 Liter zur Verwendung auf einer Kippvorrichtung bereits erfolgreich erprobt, kann aber auch bei Bioreaktoren anderer Größe angewendet werden, insbesondere bei noch größeren Bioreaktoren.

### Bezugszeichenliste

- 10: Abstandshalter
- 12: Filtermedium
- 14: Anschlussstück
- 16: Schlauchleitung
- 18: Bioreaktorwand
- 20: Durchgangsöffnung
- 22: Verbindungsabschnitt

## Patentansprüche

1. Bioreaktor zur Kultivierung von Mikroorganismen und Zellen tierischer oder pflanzlicher Herkunft, mit einer auf der Innenfläche einer flexiblen Wand (18) des Bioreaktors angeordneten Filtertasche, die außenseitig durch die Wand (18) des Bioreaktors und innenseitig durch eine Filtertaschenwand begrenzt ist, wobei die innenseitige Filtertaschenwand zumindest teilweise durch ein Filtermedium (12) gebildet ist, und wobei in der Filtertasche zwischen der Wand (18) des Bioreaktors und dem Filtermedium (12) ein Abstandshalter (10) angeordnet ist,
**dadurch gekennzeichnet, dass** der Abstandshalter (10) wenigstens eine Durchgangsöffnung (20) aufweist und ein Verbindungsabschnitt (22) des Filtermediums (12) durch die Durchgangsöffnung (20) hindurchragt und direkt mit der Wand (18) des Bioreaktors verbunden ist.

2. Bioreaktor nach Anspruch 1, **dadurch gekennzeichnet, dass** mehrere, vorzugsweise regelmäßig über den Abstandshalter (10) verteilt angeordnete, Durchgangsöffnungen (20) vorgesehen sind und mehrere Verbindungsabschnitte (22) des Filtermediums (12) durch die Durchgangsöffnungen (20) hindurchragen und direkt mit der Wand (18) des Bioreaktors verbunden sind.

3. Bioreaktor nach Anspruch 1 oder 2, **dadurch gekennzeichnet, dass** die Filtertasche frei von vollständig oder teilweise abgetrennten Kammern ist.

4. Bioreaktor nach einem der vorhergehenden Ansprüche, **dadurch gekennzeichnet, dass** die Wand (18) des Bioreaktors wenigstens teilweise aus Polyethylen gebildet ist.

5. Bioreaktor nach einem der vorhergehenden Ansprüche, **dadurch gekennzeichnet, dass** der Abstandshalter (10) zumindest teilweise aus Polyethylenterephthalat gebildet ist.

6. Bioreaktor nach einem der vorhergehenden Ansprüche, **dadurch gekennzeichnet, dass** das Filtermedium (12) eine Mikrofiltrationsmembran ist, die zumindest teilweise aus Polyethersulfon gebildet ist.

7. Bioreaktor nach Anspruch 6, **dadurch gekennzeichnet, dass** wenigstens eine der beiden Seiten der Mikrofiltrationsmembran mit einem porösen Flächengebilde verbunden ist, insbesondere einem Polypropylen/Polyethylen-Polyethylenterephthalat-Polypropylen/Polyethylen Laminat.

8. Bioreaktor nach einem der vorhergehenden Ansprüche, **dadurch gekennzeichnet, dass** in das Filtermedium (12) ein Anschlussstück (14) eingesetzt ist, an das eine Schlauchleitung (16) anschließbar ist.

9. Bioreaktor nach einem der vorhergehenden Ansprüche, **dadurch gekennzeichnet, dass** der Bioreaktor ein maximales totales Volumen von wenigstens 50 Litern, vorzugsweise wenigstens 100 Litern und weiter vorzugsweise wenigstens 200 Litern hat.

10. Verfahren zur Herstellung einer Filtertasche in einem Bioreaktor zur Kultivierung von Mikroorganismen und Zellen tierischer oder pflanzlicher Herkunft, mit folgenden Schritten:
- Bereitstellen einer flexiblen Wand (18) für die Ausbildung eines Bioreaktors, eines Abstandshalters (10) und einer Filtertaschenwand, die zumindest teilweise durch ein Filtermedium (12) gebildet ist, wobei die Filtertaschenwand eine größere Flächenausdehnung als der Abstandshalter (10) hat;
- Bilden wenigstens einer Durchgangsöffnung (20) im Abstandshalter (10), die den Abstandshalter (10) vollständig durchdringt;
- Platzieren des Abstandshalters (10) auf der Innenfläche der Wand (18) des Bioreaktors;
- Platzieren der Filtertaschenwand auf dem Abstandshalter (10);
- umlaufendes Fixieren des über den Abstandshalter (10) überstehenden Rands der Filtertasche an der Wand (18) des Bioreaktors;
- Durchführen eines Verbindungsabschnitts (22) des Filtermediums (12) durch die Durchgangsöffnung (20); und
- Fixieren des Verbindungsabschnitts (22) des Filtermediums (12) direkt an der Wand (18) des Bioreaktors.

11. Verfahren nach Anspruch 10, **dadurch gekennzeichnet, dass** das Fixieren des wenigstens einen Verbindungsabschnitts (22) des Filtermediums (12) an der Wand (18) des Bioreaktors durch Punktschweißen erfolgt.

12. Verfahren nach Anspruch 10, **dadurch gekennzeichnet, dass** das Fixieren des wenigstens einen Verbindungsabschnitts (22) des Filtermediums (12) an der Wand (18) des Bioreaktors durch Kleben erfolgt.

## Claims

1. Bioreactor for cultivating microorganisms and cells of animal or plant origin, comprising a filter pocket which is arranged on the inner surface of a flexible wall (18) of the bioreactor and which is delimited on an outside by the wall (18) of the bioreactor and on an inside by a filter pocket wall, wherein the inside filter pocket wall is formed at least partially by a filter medium (12), and wherein a spacer (10) is arranged in the filter pocket between the wall (18) of the bioreactor and the filter medium (12),
**characterized in that** the spacer (10) has at least one through-opening (20), and a connecting portion (22) of the filter medium (12) protrudes through the through-opening (20) and is directly connected to the wall (18) of the bioreactor.

2. Bioreactor according to claim 1, **characterized in that** a plurality of through-openings (20) are provided, which preferably are arranged regularly distributed across the spacer (10), and a plurality of connecting portions (22) of the filter medium (12) protrude through the through-openings (20) and are directly connected to the wall (18) of the bioreactor.

3. Bioreactor according to claim 1 or 2, **characterized in that** the filter pocket is free of completely or partially separated chambers.

4. Bioreactor according to any one of the preceding claims, **characterized in that** the wall (18) of the bioreactor is at least partially formed of polyethylene.

5. Bioreactor according to any one of the preceding claims, **characterized in that** the spacer (10) is at least partially formed of polyethylene terephthalate.

6. Bioreactor according to any one of the preceding claims, **characterized in that** the filter medium (12) is a microfiltration membrane which is at least partially formed of polyethersulfone.

7. Bioreactor according to claim 6, **characterized in that** at least one of the two sides of the microfiltration membrane is connected to a porous planar structure, in particular a polypropylene/polyethylene-polyethylene terephthalate-polypropylene/polyethylene laminate.

8. Bioreactor according to any one of the preceding claims, **characterized in that** a connecting piece (14), to which a hose line (16) can be coupled, is inserted into the filter medium (12).

9. Bioreactor according to any one of the preceding claims, **characterized in that** the bioreactor has a maximum total volume of at least 50 liters, preferably at least 100 liters, and more preferably at least 200 liters.

10. Method for producing a filter pocket in a bioreactor for cultivating microorganisms and cells of animal or plant origin, comprising the following steps:
- providing a flexible wall (18) for forming a bioreactor, a spacer (10), and a filter pocket wall formed at least partially by a filter medium (12), wherein the filter pocket wall has a greater surface area than the spacer (10);
- forming at least one through-opening (20) in the spacer (10), which passes all the way through the spacer (10);
- placing the spacer (10) on the inner surface of the wall (18) of the bioreactor;
- placing the filter pocket wall on the spacer (10);
- circumferentially fixing the edge of the filter pocket that projects beyond the spacer (10) to the wall (18) of the bioreactor;
- passing a connecting portion (22) of the filter medium (12) through the through-opening (20); and
- fixing the connecting portion (22) of the filter medium (12) directly to the wall (18) of the bioreactor.

11. Method according to claim 10, **characterized in that** the fixing of the at least one connecting portion (22) of the filter medium (12) to the wall (18) of the bioreactor takes place by spot welding.

12. Method according to claim 10, **characterized in that** the fixing of the at least one connecting portion (22) of the filter medium (12) to the wall (18) of the bioreactor takes place by adhesive bonding.

## Revendications

1. Bioréacteur pour la culture de micro-organismes et de cellules d'origine animale ou végétale, comprenant une poche de filtre disposée sur la surface intérieure d'une paroi (18) flexible du bioréacteur, qui est délimitée côté extérieur par la paroi (18) du bioréacteur et côté intérieur par une paroi de poche de filtre, dans lequel la paroi de poche de filtre côté intérieur est formée au moins en partie par un milieu filtrant (12), et dans lequel un espaceur (10) est disposé dans la poche de filtre entre la paroi (18) du bioréacteur et le milieu filtrant (12),
**caractérisé en ce que** l'espaceur (10) présente au moins une ouverture de passage (20) et une partie de liaison (22) du milieu filtrant (12) fait saillie à travers l'ouverture de passage (20) et est directement reliée à la paroi (18) du bioréacteur.

2. Bioréacteur selon la revendication 1, **caractérisé en ce que** plusieurs ouvertures de passage (20), de préférence réparties régulièrement sur toute l'étendue de l'espaceur (10), sont prévues et plusieurs parties de liaison (22) du milieu filtrant (12) font saillie à travers les ouvertures de passage (20) et sont directement reliées à la paroi (18) du bioréacteur.

3. Bioréacteur selon la revendication 1 ou 2, **caractérisé en ce que** la poche de filtre est exempte de chambres entièrement ou partiellement séparées.

4. Bioréacteur selon l'une quelconque des revendications précédentes, **caractérisé en ce que** la paroi (18) du bioréacteur est formée au moins en partie de polyéthylène.

5. Bioréacteur selon l'une quelconque des revendications précédentes, **caractérisé en ce que** l'espaceur (10) est formé au moins en partie de polyéthylène téréphthalate.

6. Bioréacteur selon l'une quelconque des revendications précédentes, **caractérisé en ce que** le milieu filtrant (12) est une membrane de microfiltration, qui est formée au moins en partie de polyéthersulfone.

7. Bioréacteur selon la revendication 6, **caractérisé en ce qu'**au moins un des deux côtés de la membrane de microfiltration est relié à une structure plane poreuse, en particulier un stratifié polypropylène/polyéthylène-polyéthylènetéréphthalate-polypropylène/polyéthylène.

8. Bioréacteur selon l'une quelconque des revendications précédentes, **caractérisé en ce qu'**une pièce de raccordement (14), à laquelle une conduite flexible (16) peut être raccordée, est insérée dans le milieu filtrant (12).

9. Bioréacteur selon l'une quelconque des revendications précédentes, **caractérisé en ce que** le bioréacteur possède un volume total maximal d'au moins 50 litres, de préférence d'au moins 100 litres et plus préférentiellement d'au moins 200 litres.

10. Procédé de fabrication d'une poche de filtre dans un bioréacteur pour la culture de micro-organismes et de cellules d'origine animale ou végétale, comprenant les étapes suivantes :
- la fourniture d'une paroi (18) flexible pour la réalisation d'un bioréacteur, d'un espaceur (10) et d'une paroi de poche de filtre, qui est formée au moins en partie par un milieu filtrant (12), dans lequel la paroi de poche de filtre possède une superficie plus grande que l'espaceur (10) ;
- la formation d'au moins une ouverture de passage (20) dans l'espaceur (10), qui traverse entièrement l'espaceur (10) ;
- le placement de l'espaceur (10) sur la surface intérieure de la paroi (18) du bioréacteur ;
- le placement de la paroi de poche de filtre sur l'espaceur (10) ;
- la fixation du bord de la poche de filtre dépassant de l'espaceur (10) sur tout le pourtour de la paroi (18) du bioréacteur ;
- le guidage d'une partie de liaison (22) du milieu filtrant (12) à travers l'ouverture de passage (20) ; et
- la fixation de la partie de liaison (22) du milieu filtrant (12) directement sur la paroi (18) du bioréacteur.

11. Procédé selon la revendication 10, **caractérisé en ce que** la fixation de l'au moins une partie de liaison (22) du milieu filtrant (12) sur la paroi (18) du bioréacteur s'effectue par soudage par points.

12. Procédé selon la revendication 10, **caractérisé en ce que** la fixation de l'au moins une partie de liaison (22) du milieu filtrant (12) sur la paroi (18) du bioréacteur s'effectue par collage.
